# EUROPEAN PATENT APPLICATION

(11) **EP 1 712 561 A1**
(43) Date of publication of application: **18.10.2006**
(21) Application number: 05703849.9
(22) Date of filing: 12.01.2005
(51) Int. Cl.: C07K 14/435, D02G 3/02, C12N 15/09, A01K 67/04, C12N 5/16

(54) **SILK THREAD CONTAINING SPIDER THREAD PROTEIN AND SILKWORM PRODUCING THE SILK THREAD**

(30) Priority: 13.01.2004 JP 2004005489
(71) Applicant: TORAY INDUSTRIES, INC., Tokyo 103-8666 (JP); E.I. DUPONT DE NEMOURS AND COMPANY, Wilmington, Delaware 19898 (US)
(72) Inventor: HIRAMATSU, Shingo, City-Heim 202, Fujisawa-shi, Kanagawa 2510013 (JP); MORIYAMA, Hiromitsu, City-Winds Mizonokuchi, Kawasaki-shi, Kanagawa 2130001 (JP); ASAOKA, Ryota, Toray Dai-ichi Urayasuryo 405, Urayasu-shi, Chiba 2790004 (JP); MORITA, Ken, Lairi ecrin 3B, Tokai-shi, Aichi 4760012 (JP); TANAKA, Takashi, Atrait Uchidabashi 1101, Nagoya-shi, Aichi 4570851 (JP); YAMADA, Katsushige, Nishikasugai-gun, Aichi 4810004 (JP); OBRIEN, John Philip, 19363 (US); FAHNESTOCK, Stephen R., 19803 (US)
(74) Representative: Denison, Christopher Marcus
(86) International application number: PCT/JP2005/000619
(87) International publication number: WO 2005/068495

(57) **Abstract**

A transgenic silkworm having transferred therein a gene which encodes spider thread protein having desired properties of high strength and high elasticity while leaving the silkworm fibroin H chain gene intact, by means of utilizing a transposon function, is used to produce in the transgenic silkworm a spider thread protein having the desired properties without lowering the strength or elasticity of silk thread produced by the transgenic silkworm, thereby providing hybrid silk of spider and silk threads having the desired properties.

## Description

### Technical Field

The present invention relates to silk thread containing spider thread protein produced by a transgenic silkworm, to the transgenic silkworm and to a method for producing silk thread from the transgenic silkworm.

### Background Art

With the ever increasing demand for materials and fabrics that are both light and flexible without lack of strength and durability, spider thread is becoming noted for its numerous desired characteristics. It is known that orb-web spider species are capable of producing silk from six different types of glandular tissue, with each of the six types of fiber exhibiting different mechanical properties. Spider threads (spider silk) produced from the major amullate gland and minor amullate gland are known as "dragline". Because dragline exhibit high tensile strength and high elasticity (Non-patent document 1), they are expected to be applicable for a wide variety of industrial uses (Non-patent document 2). Spider threads produced from the flagelliform gland and aggregate gland, which generally serve as spiral threads, exhibit high stickiness and high elasticity (Non-patent document 3).

Some such spider threads exhibit stronger tensile strength than steel and have stronger folding endurance than wool, and some are characterized by a larger energy-to-break limit than KEVLAR™. In particular, dragline exhibit tensile strength exceeding 200 ksi, with a folding endurance of about 35%. Such features offer the prospect of spider thread as a lightweight, high-strength fiber for numerous textile applications.

Moreover, since spider thread is stable up to a maximum temperature of 100°C, the fibers can be used for reinforcement of heat injection plastics. The proteins are also potentially valuable in the form of films or coatings. In addition, because spider thread is composed of protein of which amino acids are the constituent components, it is also advantageous in terms of biodegradability in the natural environment.

Known natural spider thread proteins include spidroin1 (MaSpI), spidroin2 (MaSpII) (Non-patent document 4), ADF-3, ADF-4 and the like. The characteristic sequences of these spider thread proteins comprise amino acids consisting mainly of alanine, serine and glycine, while also including considerable amounts of other amino acids such as glutamine, tyrosine, leucine and valine. The protein spidroin1 have sequence repeats, where the repeating units have the maximum length of 34 amino acids and are not highly conserved. The repeating unit comprises two different segments: (i) a 10-amino acid segment composed mostly of a 5-7 residue polyalanine sequence, and (ii) a 24-amino acid segment which is conserved in the sequence but which lacks 3 amino acids multiple times among most of the repeating units. The latter sequence consists mainly of a Gly-Xaa-Gly motif, where Xaa represents alanine, tyrosine, leucine or glutamine. The protein spidroin2 also has a repeated sequence, where the repeating unit has a maximum length of 51 amino acids and, likewise, is not highly conserved. This repeating unit comprises two different segments: (i) a segment composed mostly of a 6-9 residue polyalanine sequence, and (ii) a segment containing the amino acid sequence GlyGlyTyrGlyProGly or GlyProGlyGlnGln.

Natural spider thread proteins, and especially the main constituent protein of dragline silk, spidroin1, are known to contain polypeptides composed of glycine, serine, glutamine, alanine, tyrosine, leucine, arginine, isoleucine and valine. The sequence of the minor component, spidroin2, is known to contain more proline residues than spidroin1, and it is believed that proline contributes to the turning and twisting structure.

Artificially synthesized and designed genes that have these features are known, namely DP-1B and DP-2A (Non-patent document 4).

However, spiders are difficult to breed in groups and therefore successful production of large amounts of thread directly from spiders has been elusive. In addition, it is difficult to solubilize and purify natural spider threads, as they are insoluble without using highly caustic reagents such as LiSCN, LiClO₄ or 88% (vol/vol) formic acid. Even when dissolved, the spider thread protein of the spider threads precipitate out during dialysis or during dilution with typical buffer solutions. Furthermore, the properties of spider thread produced by spiders differs depending on the type of spider thread, and it is difficult to separate out the spider threads having the desired properties. For these reasons, it is considered impossible to obtain spider thread protein in commercially feasible amounts from natural sources at reasonable cost.

In recent years it has been attempted to modify several organisms to produce spider thread protein using transgenic techniques. For example, it has been attempted to produce spider thread proteins in microorganisms such as *E. coli* and yeast (Non-patent document 4, Patent document 1) or to use plants such as tobacco or potato (Non-patent document 5), while methods of using mammalian cells and methods of using transgenic goat for production in milk or urine have also been described (Patent document 2). However, production of spider thread protein using gene recombination requires extraction and purification of the produced spider thread protein, as well as artificial spinning (Non-patent document 1), and artificial spinning increases labor and cost while also creating an environmental burden due to the use of different solvents.

There has been disclosed a method for direct production of fibers containing spider thread protein that requires no purification and/or artificial spinning step, wherein silk thread comprising a fusion protein of green fluorescent protein and spider thread protein is produced using recombinant silkworm (Patent document 3). The method involves introducing a gene coding for a fusion protein of green fluorescent protein and spider thread protein-like protein into silkworm eggs by electroporation, and obtaining recombinant individuals by homologous recombination. However, the silk thread produced by this method has strength and elasticity that is inferior to silk thread of non-transgenic silkworms.

The following reasons for this have been proposed: 1. Gene transfer by homologous recombination results in destruction of at least one of the fibroin H chain genes of the original pair in silkworm; 2. For selection of recombinants it is necessary to express green fluorescent protein as a fusion protein, and this may impair the properties of the spider thread protein; 3. The designed spider thread protein may have a sequence that cannot exhibit the properties of high strength and high elasticity. [Patent document 1] Japanese Patent Laid-open (Kohyo) Publication HEI No. 8-511426
[Patent document 2] Japanese Patent Laid-open (Kohyo) Publication No. 2002-506642
[Patent document 3] International Patent Publication No. WO02/40528
[Non-patent document 1] Science, 2002, Vol. 295, p472-476
[Non-patent document 2] Nature, 2001, Vol. 410, p541-548
[Non-patent document 3] Osaki, S., Kumo no Ito no Kagaku, Yuki Gosei Kagaku[Spider Thread Chemistry, Organic Synthesis Chemistry] (Japan), 1985, Vol.4, No.9, p828-835.
[Non-patent document 4] Reviews in Molecular Biotechnology, 2000, No.74, p105-119
[Non-patent document 5] Nature, 2001, No.19, p573-577

### DISCLOSURE OF THE INVENTION

### Problems to be Solved by the Invention

It is an object of the present invention to provide silk thread with the desired properties of spider thread including high strength and high elasticity, without using solvents and without requiring an artificial spinning step.

### Means for Solving the Problems

As a result of diligent investigation of means for overcoming the problems described above, the present inventors discovered that by using the function of a transposon it is possible for a transgenic silkworm having a gene coding for spider thread protein transferred therein to produce silk thread containing spider thread protein that exhibits the desired properties of high strength and high elasticity, and have thereupon completed this invention.

Specifically, the invention provides the following inventions (1)-(26).
(1) Silk thread comprising spider thread protein, produced by a transgenic silkworm possessing a pair of fibroin H chain genes.
(2) Silk thread comprising spider thread protein, characterized by being produced by a transgenic silkworm possessing a pair of fibroin H chain genes and by essentially retaining the basic structure of silk thread fibroin H chain protein.
(3) Silk thread according to invention (1) or (2), characterized in that the spider thread protein is dispersed in the fibroin protein.
(4) Silk thread according to any one of inventions (1) to (3), characterized in that the spider thread protein is fused with a polypeptide contained in the fibroin H chain protein.
(5) Silk thread according to invention (4), characterized in that the spider thread protein is inserted between the N-terminal portion and C-terminal portion of the fibroin H chain protein, and is disulfide bonded with the fibroin L chain protein via a cysteine contained in the C-terminal portion.
(6) Silk thread according to any one of inventions (1) to (5), wherein the spider thread protein content is 0.1-25 wt%.
(7) Silk thread according to invention (6), wherein the spider thread protein content is 1-15 wt%.
(8) Silk thread according to invention (7), wherein the spider thread protein content is 1-10 wt%.
(9) Silk thread according to any one of inventions (1) to (8), characterized in that the spider thread protein includes the peptide listed as SEQ ID NO: 1, or the peptide listed as SEQ ID NO: 1 with a deletion, substitution or addition of one or more amino acids and having the properties of spider thread protein.
(10) Silk thread according to invention (9), characterized by comprising spider thread protein with 3-30 repeats of the peptide of invention (9).
(11) Silk thread according to invention (9), characterized by comprising spider thread protein with 4-16 repeats of the peptide of invention (9).
(12) Silk thread according to any one of inventions (1) to (8), characterized in that the spider thread protein includes the peptide listed as SEQ ID NO: 2, or the peptide listed as SEQ ID NO: 2 with a deletion, substitution or addition of one or more amino acids and having the properties of spider thread protein.
(13) Silk thread according to invention (12), characterized by comprising spider thread protein with 3-30 repeats of the peptide of invention (12).
(14) Silk thread according to invention (13), characterized by comprising spider thread protein with 4-16 repeats of the peptide of invention (12).
(15) Silk thread according to any one of inventions (1) to (8), characterized in that the spider thread protein comprises both the peptide according to invention (9) and the peptide according to invention (12).
(16) Silk thread according to any one of inventions (5) to (15), characterized in that the C-terminal portion of the fibroin H chain protein fused with the spider thread protein is the peptide of SEQ ID NO: 3 or the peptide of SEQ ID NO: 3 having a deletion, substitution or addition of one or more amino acids and having 2 or 3 cysteines.
(17) Silk thread according to any one of inventions (5) to (16), characterized in that the N-terminal portion of the fibroin H chain protein fused with the spider thread protein is the peptide of SEQ ID NO: 4 or the peptide of SEQ ID NO: 4 having a deletion, substitution or addition of one or more amino acids, and is a peptide such that the gene coding for the peptide retains the function of enhancing promoter-dependent exogenous protein expression.
(18) Silk thread according to any one of inventions (1) to (17), wherein the spider thread protein is not fused to a selection marker protein.
(19) A transgenic silkworm possessing a pair of fibroin H chain genes and producing silk thread according to any one of inventions (1) to (18) wherein the gene coding for spider thread protein is transferred into a region other than the pair of fibroin H chain genes.
(20) A transgenic silkworm according to invention (19), characterized by having a fibroin H chain gene promoter for expression of spider thread protein in the transgenic silkworm.
(21) A transgenic silkworm according to invention (19), characterized by having a fibroin H chain gene promoter and its upstream region for expression of spider thread protein in the transgenic silkworm.
(22) A transgenic silkworm according to invention (20) or (21), characterized in that the first exon·entirety or a portion of the first intron·portion of the second exon region of the fibroin H chain gene is linked downstream from the fibroin H chain promoter.
(23) A method for producing a transgenic silkworm according to any one of inventions (19) to (22), which utilizes a transposon.
(24) A method for producing a transgenic silkworm according to invention (23), characterized in that the transposon is piggyBac transposon.
(25) A method for producing silk thread characterized by using a transgenic silkworm according to any one of inventions (19) to (22).
(26) A textile employing silk thread according to any one of inventions (1) to (18).

### Effect of the Invention

The present invention provides silk thread comprising spider thread protein with excellent properties of enhanced strength, elasticity and/or toughness. Because of the excellent physical properties of textiles employing the silk thread, they have potential industrial applications that require special types of high strength, such as aeronautical and aerospace applications, clothing production, or as ropes or surgical suture thread, as well as biomaterials for transplantation (for example, artificial ligaments or aortic bandages).

### Brief Explanation of the Drawings

Fig. 1 is a diagram showing a method of constructing HP·DP16·HC, HP·DP12·HC, HP·DP8·HC, HP·DP4·HC and HP·AEX·HC gene structures.

Fig. 2 is a diagram showing a method of constructing HUP·DP8·HC and HUP·DP4·HC structures.

Fig. 3 is a photograph showing the results of Southern hybridization of restriction endonuclease EcoRI-treated genomes of randomly selected transgenic silkworms having the HP·DP8·HC (lanes 1-5) and HP·DP4·HC (lanes 6-10) gene structures, with labeling of the spider thread protein gene DP.

Fig. 4 is a set of photographs showing the results of analysis of solubilized cocoons created by silkworms having the HP·DP16·HC, HP·DP8·HC, HP·DP4·HC, HP·AEX·HC, HUP·DP8·HC and HUP·DP4·HC gene structures transferred therein, using silver staining and Western blotting with antibody.

Fig. 5 is a set of photographs showing the results of scanning electron microscopy (1500x) of the state before and after degumming of silk thread produced by a non-recombinant silkworm and silk thread produced by a recombinant silkworm having the HP·DP8·HC gene transferred therein.

Fig. 6 is a photograph (15,000x magnification) showing the results of immunoelectron microscopy conducted using anti-spider thread protein DP antibody, with silk thread produced by a non-recombinant silkworm as the sample cut into strips in the fiber axis direction.

Fig. 7 is a photograph showing the results of immunoelectron microscopy (15,000x magnification) conducted using anti-spider thread protein DP antibody, with silk thread produced by a recombinant silkworm having the HP·DP8·HC gene transferred therein as the sample cut into strips in the fiber axis direction.

### Explanation of Symbols

- 1:: Sericin layer
- 2:: Fibroin layer
- 3:: Spider thread protein

### Best Mode for Carrying Out the Invention

Other terms and concepts related to the invention will be specified in detail through the working modes and examples of the invention. The techniques used for carrying out the invention may be accomplished easily and reliably by a person skilled in the art based on the publicly available literature, except for techniques noted by specific references. For example, genetic engineering and molecular biological techniques are described in Sambrook and Maniatis, Molecular Cloning-A Laboratory Manual, Cold Spring Harbor Laboratory Press, New York, 1989; Ausubel, F. M. et al., Current Protocols in Molecular Biology, John Wiley & Sons, New York, N.Y, 1995.

"Silk thread" according to the invention refers to fiber whose major component is protein which forms the cocoon spun by the silkworm moth *(Bombyx mori).* Preferably it is raw silk thread prepared from cocoon, more preferably it is silk thread obtained by degumming of raw thread, and even more preferably it is silk thread prepared as multifilaments from two or more different cocoons. Silk thread produced by silkworm is composed of a fibroin layer and a surrounding sericin layer.

The term "spider" used according to the invention refers to an animal belonging to the order Araneae, and preferably an orb-web species belonging to the order Araneae. Most preferably, it is a cross spider or Joro spider.

The term "spider thread" according to the invention refers to thread, i.e. fibrous protein, produced by a "spider", and specifically there may be mentioned thread produced by orb-web spiders, such as dragline, frame thread, anchor thread, cocoon thread, spiral thread, swathing band, attachment thread and dragline, among which warp thread, spiral thread, frame thread and dragline are preferred.

The term "spider thread protein" according to the invention is protein composing spider thread or protein having the features of protein composing spider thread. It need not necessarily have exactly the same sequences as natural spider thread protein, and may be artificially designed and synthesized protein. Because natural spider thread protein has a huge molecular weight, the genes that produce natural spider thread protein are also huge. The genes are preferably subjected to shortening, deletion or substitution for more efficient handling. They may also be artificial genes designed so as to produce artificial protein analogues having sequences characteristic of natural spider thread protein.

The spider thread protein used is preferably protein having a sequence with 5 or more amino acid residues found in DP-1B or DP-2A. More preferably, it is patterned on the amino acid sequence of the major constituent protein spidroin1, in order to impart the silk thread with the desired strength and elastic properties of spider tractile thread. That is, spider thread protein according to the invention is preferably composed of the amino acids glycine, serine, glutamine, alanine, tyrosine, leucine, arginine, isoleucine and valine, and more preferably it is spider thread protein containing the sequence listed as SEQ ID NO: 1 encoded by DP-1B, or containing the sequence listed as SEQ ID NO: 2, comprising the "repeat characteristic of spider thread protein" + "consensus sequence of DP-2A" + "repeat characteristic of silk thread" in that order.

This also includes the sequences listed as SEQ ID NO: 1, 2, 3 or 4 having a deletion, substitution or addition of one or more amino acids, as far as the proteins have substantially equivalent function. That is, the aforementioned explanation is made with the full understanding that proteins can retain basic function even when modified by various methods. This means that, despite having mutations such as deletions, substitutions or additions of one or more amino acids with respect to the basic polypeptide sequence, the protein retains the desired properties, i.e. the basic function equivalent to that of the non-mutated sequence.

Also, in order to efficiently produce the spider thread protein using a transgenic silkworm, it is preferred to use a gene with a codon usage frequency similar to genes expressed in silkworm silk glands. As examples of genes expressed in silkworm silk glands there may be mentioned fibroin H chain gene, fibroin L chain gene and P25 protein gene. Gene modification and mutation may be carried out in order to achieve codon usage frequency similar to genes expressed in silkworm silk glands, and methods therefor are publicly known. Specifically, a method for modifying the protein-producing gene may be used. For example, random mutagenesis, site-specific mutagenesis, gene homologous recombination or polymerase chain reaction (PCR) may be used alone or in appropriate combinations. There may be employed, for example, a method of substituting uracil bases for cytosine bases by chemical treatment using sodium hydrogensulfite, a method of conducting PCR in a reaction solution containing manganese in order to lower the accuracy of nucleotide incorporation during DNA synthesis, or methods using various commercially available kits for site-specific mutagenesis. The modification may also be carried out according to or based on methods described in the literature, such as Sambrook et al., "Molecular Cloning-A Laboratory Manual, 2nd Edition", Cold Spring Harbor Laboratory, 1989; Muramatsu, M., "Laboratory Manual Genetic Engineering", Maruzen Publishing, 1988; or Erlich, HE., "PCR Technology and DNA Amplification, Principles and Applications", Stockton Press, 1989.

According to the invention, the spider thread protein is preferably fused with a polypeptide of the fibroin H chain, which is a constituent protein of silk thread. Preferably, the spider thread protein is fused with the C-terminal portion of the fibroin H chain. More preferably, the spider thread protein is inserted between the N-terminal portion of the fibroin H chain and the C-terminal portion of the fibroin H chain. The fusion protein comprising the spider thread protein constructed in this manner forms a disulfide bond with the fibroin L chain protein via a cysteine residue within the C-terminal portion of the fibroin H chain protein.

There are no particular restrictions on the molecular weight of the spider thread protein used for the invention. While a higher molecular weight is preferred to confer the desirable properties of spider thread, it can also hamper manipulation of the gene which produces the spider thread protein. Thus, the spider thread protein preferably has a molecular weight in the range of 30-300 kDa, and more preferably in the range of 30-160 kDa.

The silk thread of the invention has enhanced properties imparted by the excellent properties of the spider thread. More specifically, it exhibits one or more features selected from among properties of high tensile strength and high elasticity, high elastic modulus and excellent stretchability, and properties of excellent flexibility, a large energy vs. break limit, i.e. high toughness, and excellent adhesion, water shrinkage properties, weather resistance and light fastness, as compared to silk thread produced by non-transgenic silkworms of the same species, but this does not mean that all of these features must be satisfied. Preferably, it exhibits one or more features selected from among high tensile strength, high elasticity and high toughness.

According to the invention, the promoter used for production of the spider thread protein-containing silk thread is not particularly restricted but is preferably one with high transcription activity. As examples there may be mentioned the *Drosophila* heat shock protein gene promoter described in Japanese Unexamined Patent Publication HEI No. 6-261770 or Japanese Unexamined Patent Publication SHO No. 62-285787, or the silkworm actin gene promoter (Nature Biotechnology 18,81-84,2000), but preferred promoters are promoters with high transcription activity in silkworm silk gland cells, such as the fibroin H chain gene promoter (GenBank Accession No. V00094, nucleotides 255-574, GenBank Accession No. AF226688, nucleotides 62118-62437), fibroin L chain gene promoter (Gene, 100:151-158: GenBank Accession No. M76430), and sericin gene promoter (GenBank Accession No. AB007831, nucleotides 599-1656). High transcription promotion is also achieved by the fibroin H chain gene promoter and its upstream region (GenBank Accession No. AF226688, nucleotides 57444-62437).

The 5' terminal portion of the fibroin H chain gene is a DNA sequence having the function of enhancing expression of exogenous protein by the promoter. This DNA sequence includes the entirety or a portion of the first exon and first intron and a portion of the second exon of the fibroin H chain gene. Also, the 5' terminal portion of the fibroin H chain gene codes for a polypeptide at the N-terminal portion of the fibroin H chain protein listed as SEQ ID NO: 4.

Any insect vector capable of accomplishing gene recombination in insect cells may be used for the silkworm transformation, with no special restrictions. Examples include *Bombyx mori* nuclear polyhedrosis virus (BmNPV) vector and insect DNA transposon-incorporating plasmid vectors, with the latter being particularly preferred. As insect DNA transposons there are known piggyBac, mariner (Insect Mol. Biol. 9, 145-155, 2000) and Minos (Insect Mol. Biol. 9, 277-281, 2000), among which piggyBac is preferred for the invention.

PiggyBac transposon is a DNA transposable element having a 13 bp inverted sequence at both ends and an approximately 2.1 kbp ORF between them. There are no particular restrictions on the PiggyBac transposon used for the invention, and for example, one derived from *Trichoplusia* in cell line TN-368, *Autographa californica* NPV (AcNPV) or *Galleria mellonea* NPV (G mMNPV) may be used. Preferably, plasmids pHA3PIG and pPIGA3GFP (Nature Biotechnology 18, 81-84, 2000) comprising a portion of PiggyBac from *Trichoplusia* in cell line TN-368 are used as helper plasmids producing transposase proteins.

Because these transposons produced by helper plasmids exhibit transposable activity in silkworm cells, it is possible to transform silkworms with gene transfer vectors constructed based on these DNA transposons. In fact, a gene transfer plasmid vector constructed based on piggyBac has been successfully microinjected into silkworm eggs to achieve actual transformation of silkworms (Nat. Biotechnol. 18, 81-84 , 2000).

The properties of piggyBac may be utilized to create a transgenic silkworm by the following method, for example. First, a gene designed to produce silk thread containing spider thread protein is inserted into the genome sequence of silkworm. This may be accomplished by a method similar to the method of Tamura et al. (Nat. Biotechnol. 18, 81-84, 2000). That is, the pair of inverted repeats of piggyBac are incorporated into a suitable plasmid vector, and a gene designed to produce silk thread containing spider thread protein is inserted between the pair of inverted repeats. The gene transfer plasmid vector is then microinjected into silkworm, together with a piggyBac transposase expression vector (helper plasmid). The helper plasmid is a recombinant plasmid vector incorporating essentially only the piggyBac transposase gene region, lacking either or both of the inverted repeats of piggyBac. For the helper plasmid, the promoter used for expression of the transposase may be an endogenous transposase promoter, or it may be silkworm actin promoter or *Drosophila* HSP70 promoter, or the like. In order to facilitate the next-generation silkworm screening, a marker gene may be incorporated together into the spider thread protein expression vector which includes the gene designed to produce silk thread containing spider thread protein. In this case, a promoter sequence such as silkworm actin promoter or *Drosophila* HSP70 promoter is incorporated upstream from the marker gene, and used for expression of the marker gene.

The hatched larvae (G0 generation) from the silkworm eggs microinjected with spider thread protein expression vector are then reared. The gene designed to produce silk thread containing spider thread protein will be incorporated into some of the silkworms of the G0 generation. However, the gene designed to produce silk thread containing spider thread protein is only incorporated into a portion of the cells of the silkworm body in this generation of silkworms, and in order to obtain a silkworm wherein the gene designed to produce silk thread containing spider thread protein is incorporated into all of the cells, it is necessary to cross-breed the G0 silkworms, and obtain transgenic silkworms with the gene designed to produce silk thread containing spider thread protein being transmitted through the reproductive cells. In other words, the obtained G0 generation silkworms must be crossbred with non-recombinant silkworms, or the G0 silkworms must be crossbred with each other, and transgenic silkworms possessing the gene designed to produce silk thread containing spider thread protein selected out from among the next-generation (G1 generation) silkworms. Selection of the transgenic silkworms from the G1 generation silkworms may be accomplished using PCR or Southern blotting, for example. If the marker gene is incorporated, its phenotypic property may be used for selection. For example, if a fluorescent protein gene such as GFP is used as the marker gene, selection may be accomplished by irradiating the G1 generation silkworm eggs or larvae with excitatory light and detecting fluorescence emitted by the fluorescent protein.

The silkworms selected in this manner are transgenic silkworms incorporating into their chromosomes the gene designed to produce silk thread containing spider thread protein. Transgenic silkworms obtained in this manner differ from transgenic silkworms obtained by homologous recombination as described in WO02/40528A1 (Patent document 3), in that they have the normal silkworm fibroin H chain genes intact, i.e. they are transgenic silkworms possessing a pair of fibroin H chain genes.

According to the invention, there are no particular restrictions on the content of spider thread protein in the silk thread, and it may be in a range of 0.1%-30% based on weight. It is preferably in a range that does not impair fiber formation by the spinning behavior of the silkworm. The strength may be reduced if the content is greater than 30%. The range is more preferably 0.1-25%, even more preferably 1-15% and yet more preferably 1-10%, in order to retain strength and/or elasticity while conferring the desired properties of spider thread.

Silk thread containing spider thread protein in a weight ratio range of 0.1-25% is obtained, for example, by linking nucleotides coding for a peptide sequence comprising multiple repeats of the polypeptide listed as SEQ ID NO: 1 downstream from the fibroin H chain upstream promoter · fibroin H chain gene first exon · first intron region · portion of second exon region (GenBank Accession No. AF226688, nucleotides 57444-63513), or downstream from the fibroin H chain promoter · fibroin H chain gene first exon · first intron · second exon (GenBank Accession No. AF226688, nucleotides 62118-63513), and then further linking the fibroin H chain C-terminal region gene · fibroin H chain polyA signal region (GenBank Accession No. AF226688, nucleotides 79099-79995) downstream therefrom to produce a gene structure linked within the frame, and finally linking this to plasmid pigA3GFP treated with XhoI and then blunted. There are prepared 0.5 mM phosphate buffer (pH 7.0) · 5 mM KCl solutions containing 200 µg/ml each of this gene transfer vector and a helper plasmid containing DNA producing piggyBac transposase protein (pHA3PIG), and after egg-laying, 3-20 nl of these solutions are microinjected into the silkworm eggs within 4 hours to create transgenic silkworms. The recombinant silkworms created in this manner produce silk thread containing spider thread protein.

Differences in spider thread protein content will result due to differences in the chromosomal position of the transferred gene.

There are no particular restrictions on the number of transferred peptide sequence repeats in the spider thread protein, but it is preferably 3-30 and more preferably 4-16.

The spider thread protein content may be determined by solubilizing cocoon, subjecting it to SDS-PAGE, using a silver staining kit (Daiichi Pure Chemicals Co., Ltd.) for detection of the spider thread protein according to the manufacturer's protocol, measuring the signal strength of the result using a molecular imager (BioRad), and measuring the protein content by comparison with the signal strength for a protein of known concentration, such as feline interferon, for example.

There are no particular restrictions on the method for obtaining the DNA to be used for the invention. There may be mentioned a method of amplifying the region of the gene of interest using PCR based on known genetic data, and a method of screening with a genome library or cDNA library for homology based on known genetic data. According to the invention, the genes include those with gene polymorphisms as well as mutants obtained by artificial mutagenesis using mutagenic agents. A gene polymorphism is a partial variation in the nucleotide sequence of the gene due to natural mutation of the gene.

For secretion of spider thread protein in large quantity out of silk gland cells, the C-terminal portion of the fibroin H chain protein containing the cysteine residue which forms a disulfide bond with the fibroin L chain protein is fused with the spider thread protein to produce spider thread protein in the silk thread. The C-terminal portion of the fibroin H chain gene may be present at the N-terminal end or C-terminal end of the spider thread protein, or within the spider thread protein. At least one cysteine residue is present in this portion. The cysteine residue of the fibroin H chain protein having the role of binding with the fibroin L chain by a disulfide bond is the 20th amino acid from the C-terminal end of the fibroin H chain protein. The length of the C-terminal portion of the fibroin H chain protein which fuses with the spider thread protein is not particularly restricted so long as it does not inhibit disulfide bond formation with the fibroin L chain.

Secretion of spider thread protein in large quantity out of silk gland cells can likewise be achieved by fusion with the fibroin L chain protein containing the cysteine residue which forms a disulfide bond with the fibroin H chain protein, to allow production of spider thread protein in the silk thread. However, in order to impart the desired properties of high elasticity and high strength to the silk thread containing spider thread protein, fusion to the C-terminal portion of the fibroin H chain protein is preferred.

A "polypeptide contained in the fibroin H chain protein" according to the invention is a polypeptide at the C-terminal portion of the fibroin H chain protein containing a cysteine residue that forms a disulfide bond with the fibroin L chain protein, and/or a polypeptide encoded by the first exon and a portion of the 2nd exon of the fibroin H chain gene. A polypeptide at the C-terminal portion of the fibroin H chain protein containing a cysteine residue that forms a disulfide bond with the fibroin L chain protein is preferably the polypeptide listed as SEQ ID NO: 3. Also, a polypeptide encoded by the first exon and a portion of the 2nd exon of the fibroin H chain gene is preferably the polypeptide listed as SEQ ID NO: 4. Such polypeptides may have a deletion, substitution or addition of one or more amino acids, as far as the polypeptide produces substantially equivalent function.

There are no particular restrictions on the polyA addition region, but it is preferably the polyA addition region of a protein gene that is expressed in large amounts by silk glands, such as the fibroin H chain, the fibroin L chain and sericin.

A transgenic silkworm according to the invention is a silkworm having a gene structure designed to produce silk thread containing spider thread protein. In order to facilitate selection of recombinants, a marker gene such as an antibiotic resistance gene or jellyfish-derived green fluorescent protein gene may be incorporated therein.

Jellyfish-derived green fluorescent protein (GFP) according to the invention is green fluorescent protein from *Aequorea coerulescens,* and it is characterized by emitting green fluorescence in response to excitatory light. There may also be used analogues having modified fluorescent intensity or fluorescent wavelength.

However, in order to exhibit the high-strength and high-elasticity features of spider thread protein, it is not desirable for the gene coding for the selection marker protein to be in continuous frame with the gene coding for the spider thread protein, thereby expressing a fusion protein with the selection marker protein. This is because fusion of low-crystalline, non-fibrous GFP with spider thread protein which is a highly crystalline fibrous protein can inhibit crystallization of the silk thread and lead to reduced cocoon weight and reduced silk thread strength. Thus, the stipulation that "the spider thread protein is not fused to a selection marker protein" is an important aspect to avoid reduction in strength, elasticity and/or toughness, in contrast to the examples disclosed in WO02/40528 (Patent document 3). The method described in WO02/40528 (Patent document 3) essentially requires fusion with a selection marker protein such as GFP, but according to the invention there is no need for fusion with a selection marker protein.

The expression "transgenic silkworms possessing a pair of fibroin H chain genes" according to the invention refers to transgenic silkworms that have a normal fibroin H chain gene, without exogenous genes such as the spider thread protein-encoding gene or GFP fluorescent protein gene inserted in the fibroin H chain gene, and that have an exogenous gene that is non-native to silkworm in a region other than the fibroin H chain gene. Preferably, it is a transgenic silkworm having the pair of fibroin H chain genes at the same gene locus on homologous chromosomes.

In order to confirm that the exogenous gene is transferred elsewhere than the pair of fibroin H chain genes, first a fragment from the transgenic silkworm genome having the exogenous gene inserted therein is obtained by techniques employed for ordinary cloning, using inverse PCR or the Southern hybridization method using the silkworm genome treated with various restriction endonucleases and using the exogenous gene as the probe. Confirmation of the base sequence of the obtained fragment may be accomplished by verifying that the exogenous gene has been inserted elsewhere than at the fibroin H chain gene (GenBank Accession No. AF226688, nucleotides 57444-79995).

Silkworm silk thread is produced by fiber formation primarily by fibroin H chain protein. Thus, having a pair of fibroin H chain genes is important for avoiding reduction in physical properties such as strength and elasticity of the silk thread produced by the transgenic silkworm.

According to the invention, retaining the basic structure of fibroin H chain protein means that the structure of the fibroin layer having fibroin H chain protein as the principal constituent component is a similar morphology to the fibroin layer of silk thread spun by a non-transgenic silkworm, and this can be confirmed by optical or electron microscope observation. Substantial retention of the structure of fibroin H chain protein is also important for avoiding reduction in physical properties such as strength and elasticity of the silk thread produced by the transgenic silkworm.

When these silkworms are crossbred with non-transgenic silkworms or with other transgenic silkworms, the gene designed to produce silk thread containing spider thread protein is not lost but is inherited by progeny, thereby propagating to successive generations the ability to produce silk thread containing spider thread protein.

Thus, by successive breeding of recombinant silkworms to increase the head count, it is possible to easily scale-up the production volume of silk thread containing spider thread protein. Crossbreeding between commercial silkworm breeds can also improve the production volume of silk thread containing spider thread protein. Such crossbreeding will require successive breeding with appropriate selection of silkworms having transferred therein the target gene designed to produce silk thread containing spider thread protein. In this case, DNA from cells obtained from selected tissue may be used for PCR and Southern blotting to analyze the presence and structure of the marker gene used for recombinant silkworm selection and the gene designed to produce silk thread containing spider thread protein, thereby allowing easy discrimination of progeny that have inherited the recombinant silkworm gene. When a fluorescent protein gene such as GFP is used, selection may be accomplished by irradiating the silkworm eggs or larvae with excitatory light and detecting the fluorescence emitted by the fluorescent protein.

Microinjection is a suitable method for transferring genes into cells of *Bombyx mori* eggs. Microinjection into eggs does not necessarily need to be direct microinjection into the egg cells, as transfer of the gene can be achieved by simple microinjection into the egg.

The expression "strength is improved compared to silk thread produced by non-transgenic silkworms of the same species" according to the invention means that the desired property of the high strength characteristic of spider thread is reflected. Specifically, this means improvement in the breaking strength at which breakage occurs when a load is applied to the silk thread.

The strength may be measured using a tensile tester (Tensilon). The breaking strength is represented by the maximum point load to breaking (g) divided by the thread size (d). The thread size may be measured by calculation from the weight of a unit length. Judgment on improvement or lack of improvement can be made by comparing the strength of silk thread obtained from a cocoon produced by the transgenic silkworm with that of silk thread obtained from a cocoon produced by a non-transgenic silkworm of the same species. The strength is preferably improved by at least 1% compared to silk thread produced by a non-transgenic silkworm of the same species. More preferably, the strength is improved by at least 10% and even more preferably by at least 20%.

The expression "elasticity is improved compared to silk thread produced by non-transgenic silkworms of the same species" according to the invention means that the desired property of the high elasticity characteristic of spider thread is reflected. Specifically, this means improvement in the breaking elongation at which breakage occurs when a load is applied to the silk thread. The elasticity may be measured using a tensile tester (Tensilon). Judgment on improvement or lack of improvement can be made by comparing the breaking elongation of silk thread obtained from a cocoon produced by the transgenic silkworm with that of silk thread obtained from a cocoon produced by a non-transgenic silkworm of the same species. The elasticity is preferably improved by at least 1% compared to silk thread produced by a non-transgenic silkworm of the same species. More preferably, the elasticity is improved by at least 10% and even more preferably by at least 30%.

The toughness, calculated as strength x (elasticity)^{1/2}, is preferably improved by at least 1% compared to silk thread produced by a non-transgenic silkworm of the same species. More preferably, the toughness is improved by at least 10% and even more preferably by at least 40%.

The expression "textile employing silk thread containing spider thread protein" according to the invention refers to a textile wherein spider thread protein is used for warp and/or weft thread. It may be a textile composed only of silk thread containing spider thread protein, it may employ blended thread comprising non-transgenic silkworm silk thread or blended thread comprising non-silk thread fiber, or it may be used as a processed surface-treated fabric or processed nonwoven fabric. Alternatively, it may be used as a knitted fabric (knit). The fabric-forming method is not particularly restricted, and specifically there may be mentioned plain weaving, twill weaving, satin weaving and leno weaving.

The existence form of the spider thread protein in the silk thread is predicted to be dispersed and localized. "Localized" means that the recombinant protein is partially aggregated in the fibroin layer. When localized, electron microscope observation by double staining with uranyl acetate and lead shows regions with different dye affinity. Alternatively, a slice prepared from silk thread fixed with glutaraldehyde and embedded in an epoxy resin may be observed by immunoelectron microscopy using antibody for the recombinant protein, allowing judgment based on local existence of the recombinant protein-originating signal in the fibroin layer. When the recombinant protein is dispersed in the silk thread, immunoelectron microscopy observation can likewise show its presence without local existence of the recombinant protein-originating signal in the fibroin layer.

The silk thread of the invention characterized by comprising spider thread protein dispersed in the silk thread is an alloy of different proteins (polymers), composed of the fibroin protein constituent of silk thread and spider thread protein. Most blends of two different polymers result in separation of the two due to their different properties, and reduction of their respective properties. In the case of silk thread, blending of the fibroin protein constituent of silk thread and a recombinant protein other than fibroin and artificially forming fibers fails to produce uniform blending of the fibroin protein and recombinant protein due to differences in their crystallinities, thereby reducing the physical properties including strength and elasticity. The method disclosed by the present invention, however, allows uniform blending of the fibroin protein constituent of silk thread and a recombinant protein other than fibroin, to obtain recombinant protein-containing silk thread characterized by having the recombinant protein dispersed in the silk thread. This method for producing silk thread permits the desired properties of the recombinant protein to be imparted to silk thread without lowering the strength and elasticity of fibroin.

The present invention will now be explained in greater detail through the following examples, with the understanding that the invention is in no way limited by the descriptions in the examples.

### Examples

### [Example 1] Preparation of Bombyx mori genomic DNA

Fifth-instar-three-day-old silkworms were dissected and the posterior silk gland tissue was removed. After rinsing with 1xSSC, 200 µl of DNA extraction buffer (50 mM Tris-HCl pH 8.0, 1 mM EDTA pH 8.0, 100 mM NaCl) was added. Proteinase K (final concentration: 200 µg/ml) was added and the tissue was thoroughly crushed with a grinder, after which 350 µl of DNA extraction buffer and 60 µl of 10% SDS were added prior to mixing. The mixture was incubated at 50°C for 2 hours. After then adding 500 µl of Tris-HCl equilibrated phenol (pH 8.0) and stirring for 10 minutes, centrifugation was performed at 10,000 rpm for 5 minutes at 4°C and the supernatant was collected. The supernatant was treated with phenol/chloroform and the genomic DNA was ethanol precipitated. It was then dissolved and diluted to 100 µg/ml with RNase-added sterilized water to prepare a genomic DNA solution.

### [Example 2] Gene preparation

The gene used may be obtained by PCR with end primers prepared utilizing known sequences and a suitable DNA source used as template. Restriction endonuclease cleavage sites are attached to the ends of the primers for subsequent gene manipulation.

The fibroin H chain promoter · fibroin H chain gene first exon · first intron · second exon region (GenBank Accession No. AF226688, nucleotides 62118-63513: hereinafter referred to as HP region) is obtained by PCR using *Bombyx mori* genomic DNA as template and two different primers, Primer 5 (SEQ ID NO: 5) and Primer 6 (SEQ ID NO: 6).

The fibroin H chain upstream promoter · fibroin H chain gene first exon · first intron region (GenBank Accession No. AF226688, nucleotides 57444-62927: hereinafter referred to as HUP region) is obtained by PCR using *Bombyx mori* genomic DNA as template and two different primers, Primer 7 (SEQ ID NO: 7) and Primer 8 (SEQ ID NO: 8).

The fibroin H chain C-terminal region gene · fibroin H chain gene polyA signal region (GenBank Accession No. AF226688, nucleotides 79099-79995: hereinafter referred to as HC region) is obtained by PCR using *Bombyx mori* genomic DNA as template and two different primers, Primer 9 (SEQ ID NO: 9) and Primer 10 (SEQ ID NO: 10).

PCR was carried out using KODplus (Toyobo, Ltd.) according to the manufacturer's protocol. Specifically, when *Bombyx mori* genomic DNA is used as template, 100 ng thereof is added, and the reagents are added at 50 pmol for each primer, 10 µl of the included 10×PCR buffer, and 1 mM MgCl₂, 0.2 mM dNTPs and 2 units of KODplus, to a total of 100 µl. A DNA thermal cycler by Perkin-Elmer was used with DNA denaturing conditions of 94°C, 15 sec, primer annealing conditions of 55°C, 30 sec and extension conditions of 68°C, 60 sec-300 sec, with 30 cycles of reaction.

Each reaction solution was electrophoresed on 1% agarose gel, and ordinary methods were used for extraction and preparation of a DNA fragment of approximately 1.4 kbp in the HP region, approximately 5.5 kbp in the HUP region and approximately 0.8 bp in the HC region. The DNA fragments were phosphorylated with polynucleotide kinase (product of Takara Shuzo) and then cut with BamHI and blunted by an established method using T4DNA polymerase, and ligated with dephosphorylated pUC19 vector using a DNA Ligation Kit Ver.2 by Takara Shuzo, with reaction overnight at 16°C. *E. coli* was transformed using the vectors according to ordinary methods, and insertion of the PCR fragments into the obtained transformants was confirmed by PCR of the obtained colonies under the same conditions described above, upon which the PCR fragment-inserted plasmids were prepared out by an ordinary method. The plasmids were sequenced to confirm that the obtained fragments had the respective gene nucleotide sequences.

Spider thread genes are available as the following strains from ATCC: *E. coli* FP3227 69326, 6/15/1993, *E. coli* FP 2193 69327, 6/15/1993, *E. coli* FP 3350 69328, 6/15/1993.

Alternatively, they may be constructed based on artificially synthesized oligonucleotides according to the methods disclosed in Reviews in Molecular Biology 74 (2000), 105-109 and Appl. Microbiol. Biotechnol. 47 (1997), 23-39. Specifically, BglII and BamHI restriction endonuclease recognition sites, designed to be in the same frame, may be successively linked at both ends of artificially synthesized oligonucleotides to create spider thread genes of different sizes. In this example, there were prepared DP4 having 4 repeats of polypeptide DP-1B.33 listed as SEQ ID NO: 1, DP8 having 8 repeats of DP-1B.33, DP12 having 12 repeats of DP-1B.33 and DP16 having 16 repeats of DP-1B.33.

By the same method, oligonucleotides coding for the polypeptide listed as SEQ ID NO: 2 were prepared and linked together to construct a plasmid coding for a protein comprising 8 repeats of the polypeptide listed as SEQ ID NO: 2, and this was cleaved with BglII and BamHI to construct an AEX gene fragment.

### [Example 3] Preparation of gene transfer plasmid

The gene transfer plasmid used was pigA3GFP (Nature Biotechnology 18,81-84, 2000). Specifically, pigA3GFP is a vector obtained by removing the transposase-coding region from plasmid p3E1.2 disclosed in U.S. Patent No. 6218185, and inserting at that section the A3 promoter (GenBank Accession No. U49854, nucleotides 1764-2595) and pEGFP-N1 vector (Clontech)-derived GFP and SV40 polyA addition sequence (GenBank Accession No. U55762, nucleotides 659-2578); this vector may be dispensed from the National Institute of Agrobiological Sciences. The XhoI site upstream from the A3 promoter was blunted and the gene designed to produce silk thread containing spider thread protein was inserted.

The structures of the genes designed to produce silk thread containing spider thread protein in this example were HP·DP16,12,8,4·HC and HP·AEX·HC, as well as HUP·DP8, 4·HC.

The method is described specifically below.

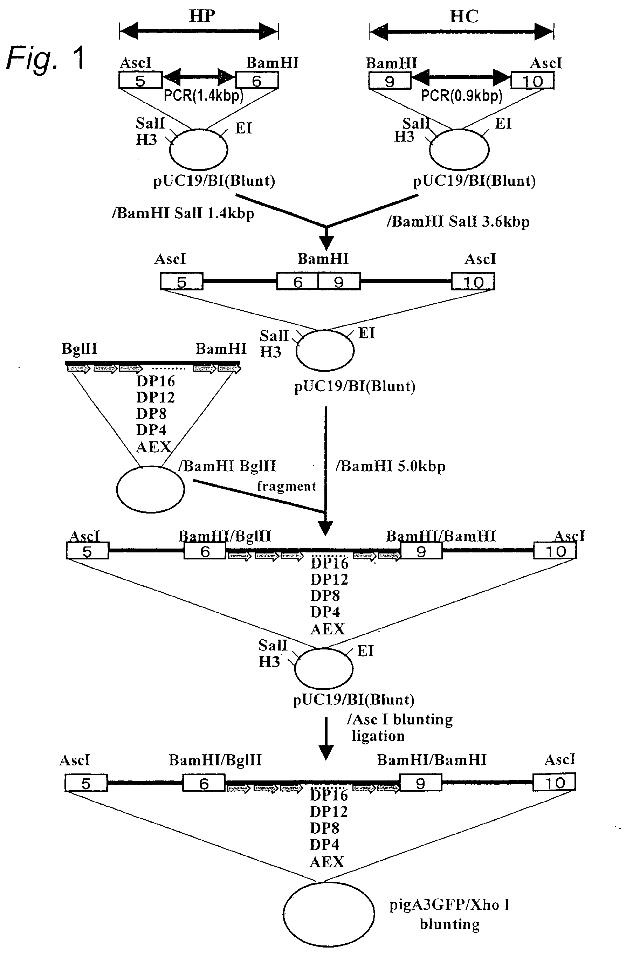

Fig. 1 shows a construction strategy for HP·DP16, 12,8,4·HC and HP·AEX·HC. The plasmid possessing the HP region and the plasmid possessing the HC region prepared in Example 2 were treated with BamHI and SalI, and the approximately 1.4 kbp and 3.6 kbp fragments were ligated. This was treated with BamHI, and the dephosphorylated plasmid was ligated with the DP16 fragment, DP12 fragment, DP8 fragment, DP4 fragment or AEX fragment having BglII and BamHI-attached ends, obtained in Example 2. The base sequences of the ligated sections were confirmed, and it was also verified that the ligated sections were in the same reading frame and properly oriented. Each plasmid was treated with AscI, and each DNA fragment containing a gene coding for spider thread protein was ligated with plasmid pigA3GFP that had been treated wih XhoI and then blunted, to construct different gene-transfer vectors comprising the gene structures HP·DP16·HC, HP·DP12·HC, HP·DP8·HC, HP·DP4·HC and HP·AEX·HC.

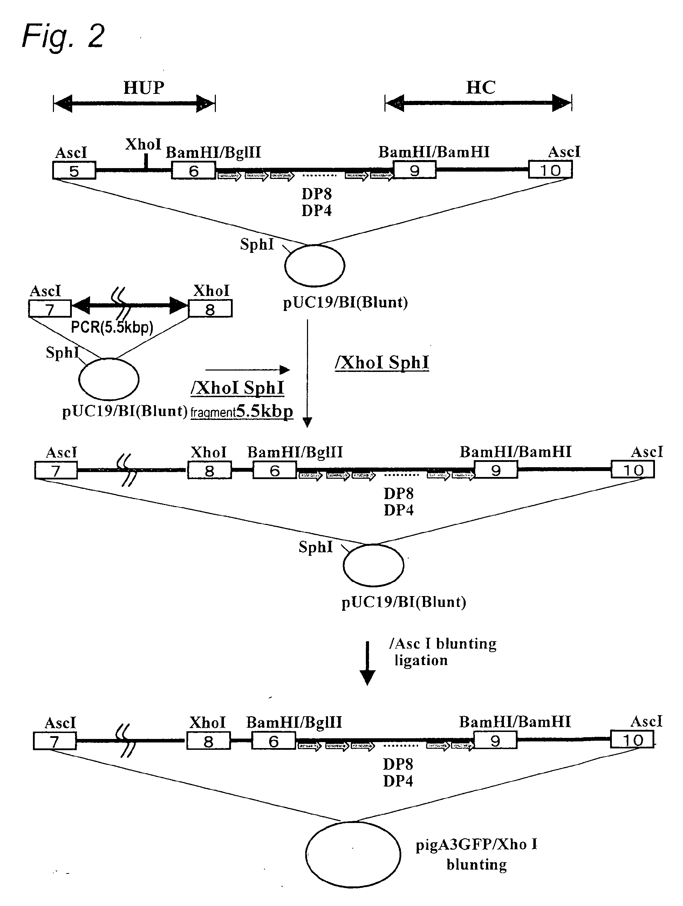

Fig. 2 shows a construction strategy for HUP·DP8, 4·HC. The HUP region-containing plasmid prepared in Example 2 was treated with SphI and XhoI to obtain an approximately 5.5 kbp fragment. The plasmids obtained by the procedure of constructing HP·DP8·HC and HP·DP4·HC were ligated with the fragment obtained by treatment with SphI and XhoI, as shown in Fig. 2. The base sequence of the ligated section was confirmed, and it was also verified that no deletions or insertions of bases had occurred. Each plasmid was treated with AscI, and each DNA fragment containing a gene coding for spider thread protein was ligated with plasmid pigA3GFP that had been treated wih XhoI and then blunted, to construct different gene-transfer vectors comprising the gene structures HUP·DP8·HC and HUP·DP4·HC.

### [Example 4] Generation of transgenic silkworms

A 0.5 mM phosphate buffer (pH 7.0), 5 mM KCl solution was prepared containing 200 µg/ml each of the gene transfer vector described in Example 3 and the helper plasmid DNA (pHA3PIG) producing piggyBac transposase protein (Nature Biotechnology 18, 81-84, 2000, dispensed from the National Institute of Agrobiological Sciences), and after egg-laying, 3-20 nl of these solutions were microinjected into 500 silkworm eggs within 4 hours. Plasmid pHA3PIG lacks one of the inverted repeats of the piggyBac transposon, its 5' flanking region and the transposase gene reader sequence, instead incorporating the 5' flanking region and reader sequence of the silkworm actin gene. Plasmid pHA3PIG has the function of producing piggyBac transposase protein under control of the actin promoter, but because it lacks one of the inverted repeats of the piggyBac transposon, its own DNA is not transferred. Larvae hatched from eggs of this silkworm were raised, and upon crossing the resulting adults (G0) within the group, the next generation (G1) was observed for fluorescence by jellyfish green fluorescent protein (GFP) in order to screen silkworms having the jellyfish green fluorescent protein gene transferred into their chromosomes. As a result, transgenic silkworms were obtained which emitted fluorescence due to the jellyfish green fluorescent protein. Selection of transgenic individuals was accomplished by screening the G1 larvae obtained by internal crossbreeding based on GFP fluorescence. Gene expression under the control of actin promoter in the posterior silk gland tissue was extremely low, and no binding occurred with fibroin L chain protein which is essential for secretion from the silk gland tissue into silk thread. That is, according to the invention GFP is not included in the silk thread containing spider thread protein, and therefore fluorescence by GFP in the cocoon and silk thread is not used as an index for selection of transgenic silkworms. The results of creating transgenic silkworms are shown in Table 1.
[Table 1]

**Table 1 Results of transgenic silkworm creation**

| Gene structure | Number of eggs injected | Number of eggs hatched | Total number G 1 | Number of GFP-positive G 1 | Transformation rate % |
|---|---|---|---|---|---|
| HP·DP16·HC | 500 | 80 | 32 | 3 | 4.7 |
| HP·DP12·HC | 1056 | 169 | 51 | 8 | 7.8 |
| HP·DP8·HC | 500 | 202 | 93 | 14 | 7.1 |
| HP·DP4·HC | 500 | 171 | 81 | 13 | 8.2 |
| HP·AEX·HC | 500 | 211 | 96 | 15 | 7.8 |
| HUP·DP8·HC | 1824 | 544 | 170 | 4 | 1.2 |
| HUP·DP4·HC | 1152 | 414 | 139 | 7 | 2.5 |

### [Example 5] Confirmation of spider thread protein gene transfer by Southern hybridization

GFP fluorescence-exhibiting silkworms were randomly selected from among the G1 silkworms having the HP·DP8·HC, HP·DP4·HC genes transferred therein, and following the method described in Example 1, genomic DNA was prepared, restriction endonuclease treatment was carried out with EcoRI, and blotted to the membrane after electrophoresis. A probe was used for labeling the DP8/BglII, BamHI fragments with a Gene-Image™ Random prime labeling and detection module by Amersham-Pharmacia. The gene coding for spider thread protein was detected according to the manufacturer's protocol. The results are shown in Fig. 3. A gene coding for spider thread protein was detected in all of the GFP fluorescence-exhibiting silkworms examined (indicated by arrows). The differences in signal sizes suggested that insertion was random on the chromosomes.

### [Example 6] Analysis of cocoons produced by transgenic silkworms

Detection of the spider thread protein in the silk thread and measurement of its content were carried out in the following manner.
After measuring out 10 mg each of cocoons of a non-transgenic silkworm (WT) and transgenic silkworms (HP·DP16·HC, HP·DP12·HC, HP·DP8·HC, HP·DP4·HC, HP·AEX·HC, HUP·DP8·HC, HUP·DP4·HC gene-transferred silkworms), adding 4 ml of 60% LiSCN and stirring, the mixtures were allowed to stand at room temperature overnight for dissolution of the cocoons. Each was diluted 10-fold with 8 M urea/2% SDS/5% 2-mercaptoethanol to prepare a sample which was subjected to SDS-PAGE, after which the spider thread protein was detected using a silver staining kit (Daiichi Pure Chemicals Co., Ltd.), according to the manufacturer's protocol. The signal strength was measured using a molecular imager (BioRad) and was compared with the signal strength using feline interferon as a protein of known concentration, to determine the protein content. An FXPro Molecular Imager by BioRad was used for signal comparison.

Also, an ECL Plus™ Western blotting Kit (Amersham-Pharmacia) was used for detection of the spider thread protein according to the manufacturer's protocol. Rabbits were used to prepare specific peptide antibody with a sufficient antibody titer for the peptide CGAGQGGYGGLGSQAGRG listed as SEQ ID NO: 11 for detection of DP and specific peptide antibody with a sufficient antibody titer for the peptide CGPGQQGPGGYGPGQQGPS listed as SEQ ID NO: 12 for detection of AEX, and these antibodies were used for detection according to the manufacturer's protocol. Specifically, the blotted membrane was subjected to blocking overnight at 4°C in blocking solution (5% skim milk/0.1% Tween20/PBS). The membrane was rinsed twice with TPBS (0.1% Tween20/PBS), and treated for one hour at room temperature with anti-spider thread protein antibody diluted 1000-fold with TPBS. The membrane was then rinsed twice with TPBS and further rinsed three times with TPBS for 5 minutes. After subsequent 10,000-fold dilution with TPBS, it was treated for one hour at room temperature using HRP-labeled anti-rabbit IgG antibody. The membrane was then rinsed twice with TPBS and further rinsed three times with TPBS for 5 minutes, after which the detection reagent (solution A + solution B) of an ECL Plus™ Western blotting Detection System (Amersham-Pharmacia) was added. Light emission was exposed on a Hyperfilm TMECLTM and developed. Fig. 4 shows the results of analysis of cocoons produced by silkworms having the gene structures HP·DP16·HC, HP·DP8·HC, HP·DP4·HC, HP·AEX·HC, HUP·DP8·HC and HUP·DP4·HC transferred therein. The molecular weights of the spider thread proteins matched the molecular weights predicted from the gene structures. The proportion of spider thread protein was approximately 0.1%-15% based on weight. There were also detected signals (indicated by asterisk) for disulfide-bonding with fibroin L chain protein, having increased size corresponding to the molecular weight of fibroin L chain protein. The HUP gene structures (HUP·DP8·HC, HUP·DP4·HC) had increased spider thread protein contents compared to the HP-gene structures (HP·DP8·HC, HP·DP4·HC). The presence of GFP in the silk thread was examined by Western analysis using antibody for GFP (Cosmo Bio Co., Ltd.), but no GFP was detected.

The average weights of cocoons (cocoons + pupae) produced by silkworms having HP·DP16·HC, HP·DP12·HC, HP·DP8·HC, HP·DP4·HC, HP·AEX·HC, HUP·DP8·HC and HUP·DP4·HC gene structures transferred therein were measured and compared using 10 male and 10 female cocoon grains each. The spider thread protein contents (wt%) were also measured. The results are shown in Table 2. No major changes in cocoon weight were seen, except for HP·AEX·HC. This indicated that DP spider thread protein did not inhibit fiber formation of silkworm fibroin. It is believed that gene transfer being accomplished not by homologous recombinant but by transposon resulted in lack of mutation in the silkworm fibroin H chain gene.
[Table 2]

**Table 2**

| Gene structure | Average weight (mg) | Spider thread protein content (%) |
|---|---|---|
| Non-transgenic | 100 | 0 |
| HP·DP16·HC | 94 | 0.5 |
| HP·DP12·HC | 104 | 2 |
| HP·DP8·HC | 104 | 0.5-5 |
| HP·DP4·HC | 102 | 1-3 |
| HUP·DP8·HC | 77 | 4-18 |
| HUP·DP4·HC | 85 | 5-15 |
| HP·AEX·HC | 34 | 0.5-1 |

### [Example 7] Preparation of silk thread

Cocoon produced by a non-transgenic silkworm as a control and cocoon produced by a silkworm having the HP·DPS·HC gene structure transferred therein as a sample were dried at 80°C to a weight of 40%. Cocoon cooking was performed in a 3% Na₂CO₃ solution at 95°C for 2 minutes, 60°C for 3 minutes and 95°C for 3 minutes. After rinsing with hot water at 40°C, a hand-reeling machine (Saito Kiryo) was used for thread reeling from 40 ordinary cocoon grains. Approximately 1100 m of non-transgenic silkworm silk thread was obtained, and approximately 1200 m of transgenic silkworm (HP·DP8·HC) silk thread was obtained.

The obtained silk thread was heated to 60°C in a 0.1% Na₂CO₃ solution, and then boiled for one hour in a 1% aqueous marseilles soap solution, washed with a 0.1% Na₂CO₃ solution and rinsed with water for degumming of the silk thread. It was then dried at 60°C to obtain degummed non-transgenic silk thread and degummed HP·DP8·HC silk thread. Fig. 5 shows a scanning electron microscope photograph (1500x) of the silk thread prior to degumming and the silk thread after degumming. Removal of the surface sericin is shown. Upon comparing the weights before and after degumming, a degumming reduction of 16% was found for both the non-transgenic silk thread and the HP·DP8·HC silk thread, suggesting that the sericin had been removed.

### [Example 8] Measurement of silk thread properties

For measurement of the physical properties there were used non-transgenic silk worm silk thread and silk thread produced by silkworm having the HP·DP8·HC gene structure transferred therein (HP·DP8·HC), prepared by the method described in Example 7, as silk thread containing spider thread protein, and the physical properties of each were measured before and after degumming. The spider thread protein content of the silk thread was 5% in HP·DP8·HC and 0% in the non-transgenic silk thread.

Measurement of the thread size was calculated based on the weight per 90 m. A tensile tester (Tensilon) was used for measurement of the mechanical properties, with calculation from the average of ten specimens each using a length of specimen between grips of 20 cm and a pull speed of 20 cm/min. Table 3 shows a comparison of the physical properties.
[Table 3]

**Table 3**

| | Non-transgenic silk thread (before degumming) | HP·DP8·HC silk thread (before degumming) | Non-transgenic silk thread (after degumming) | HP·DP8·HC silk thread (after degumming) |
|---|---|---|---|---|
| Thread size (d) | 57.7 | 87.7 | 43 | 56 |
| Strength (g/d) | 3.78 | 3.93 | 2.59 | 3.53 |
| Elasticity (%) | 32.6 | 37.8 | 13.2 | 19.1 |
| Toughness* | 21.5 | 24.1 | 9.4 | 15.4 |

| | | | | |
|---|---|---|---|---|
| *The toughness was calculated as strength x (elasticity)^{1/2}. | | | | |

For all of the properties tested, the HP·DP8·HC silk thread had superior physical properties to the non-transgenic silk thread. In particular, even after heat treatment for degumming, the strength was improved by 30% or greater, the elasticity was improved by 40% or greater and the toughness was improved by 60% or greater. This is attributed to the effect of the spider thread protein in the fibroin layer which remains even after degumming.

### [Example 9] Production of woven fabric using silk thread containing spider thread protein

A fabric was prepared using HP·DP8·HC silk thread. Two HP·DP8·HC silk threads were twill woven with a bias of 200 T/m to produce a woven fabric.

### [Example 10] Immunoelectron microscopy

Immunoelectron microscopy was carried out using cocoons produced by a transgenic silkworm having the HP·DP8·HC gene transferred therein and by a non-transgenic silkworm as samples, and using specific peptide antibody (DP antibody) having a sufficient antibody titer for the oligopeptide CGAGQGGYGGLGSQAGRG. Each sample was fixed for one hour at room temperature using 2% glutaraldehyde-0.1% cacodylate buffer, and then the epoxy resin EPON812 was used for polymerization and embedding at 60°C for 48 hours. Ultrathin slices were prepared with an ultramicrotome. Blocking was performed at room temperature for 30 minutes using 1% BSA/PBS + 1.5% goat serum. DP antibody diluted 5000-fold was used as the primary antibody, for antibody treatment overnight at 4°C. Rinsing was then carried out three times at room temperature for 10 minutes using 1% BSA/PBS. Anti-rabbit IgG Goat-Poly 10 nm (product of British Biocell International, Ltd.) was used as the secondary antibody for treatment at room temperature for one hour. Rinsing was then carried out three times at room temperature for 10 minutes using 1% BSA/PBS. Double staining was performed at room temperature for 5 minutes each with uranyl acetate and lead, prior to electron microscope observation. Fig. 6 shows the results using a longitudinal slice of silk thread produced by non-transgenic silkworm. The presence of spider thread protein in the silk thread fibroin layer and sericin layer could not be confirmed. Fig. 7 shows the results using a longitudinal slice of silk thread produced by a transgenic silkworm having the HP·DP8·HC gene transferred therein. In the longitudinal slice of silk thread produced by a transgenic silkworm having the HP·DPS·HC gene transferred therein, the presence of dispersed spider thread protein can be seen as black dots in the silk thread fibroin layer. No partially local existence is observed.

### Industrial Applicability

The hybrid silk composed of spider thread and silk thread produced according to the invention retains the high-strength and high-elasticity properties of spider thread protein, and therefore has numerous potential industrial applications, including aeronautical and aerospace development, clothing production, and uses as ropes or medical thread.

## Claims

1. Silk thread containing spider thread protein, **characterized by** being produced by a transgenic silkworm possessing a pair of fibroin H chain genes.

2. Silk thread containing spider thread protein according to claim 1, **characterized in that** said silk thread essentially retains the basic structure of silk thread fibroin H chain protein.

3. Silk thread according to claim 1 or 2, **characterized in that** the spider thread protein is dispersed in the fibroin protein.

4. Silk thread according to any one of claims 1 to 3, **characterized in that** the spider thread protein is fused with a polypeptide contained in the fibroin H chain protein.

5. Silk thread according to claim 4, **characterized in that** the spider thread protein is inserted between the N-terminal portion and C-terminal portion of the fibroin H chain protein, and is disulfide bonded with the fibroin L chain protein via a cysteine contained in the C-terminal portion.

6. Silk thread according to any one of claims 1 to 5, wherein the spider thread protein content is 0.1-25 wt%.

7. Silk thread according to claim 6, wherein the spider thread protein content is 1-15 wt%.

8. Silk thread according to claim 7, wherein the spider thread protein content is 1-10 wt%.

9. Silk thread according to any one of claims 1 to 8, **characterized in that** the spider thread protein includes the peptide listed as SEQ ID NO: 1, or the peptide listed as SEQ ID NO: 1 with a deletion, substitution or addition of one or more amino acids and having the properties of spider thread protein.

10. Silk thread according to claim 9, **characterized by** comprising spider thread protein with 3-30 repeats of the peptide of claim 9.

11. Silk thread according to claim 10, **characterized by** comprising spider thread protein with 4-16 repeats of the peptide of claim 9.

12. Silk thread according to any one of claims 1 to 8, **characterized in that** the spider thread protein includes the peptide listed as SEQ ID NO: 2, or the peptide listed as SEQ ID NO: 2 with a deletion, substitution or addition of one or more amino acids and having the properties of spider thread protein.

13. Silk thread according to claim 12, **characterized by** comprising spider thread protein with 3-30 repeats of the peptide of claim 12.

14. Silk thread according to claim 13, **characterized by** comprising spider thread protein with 4-16 repeats of the peptide of claim 12.

15. Silk thread according to any one of claims 1 to 8, **characterized in that** the spider thread protein contains both the peptide according to claim 9 and the peptide according to claim 12.

16. Silk thread according to any one of claims 5 to 15, **characterized in that** the C-terminal portion of the fibroin H chain protein fused with the spider thread protein is the peptide of SEQ ID NO: 3 or the peptide of SEQ ID NO: 3 having a deletion, substitution or addition of one or more amino acids and having 2 or 3 cysteines.

17. Silk thread according to any one of claims 5-16, **characterized in that** the N-terminal portion of the fibroin H chain protein fused with the spider thread protein is the peptide of SEQ ID NO: 4 or the peptide of SEQ ID NO: 4 having a deletion, substitution or addition of one or more amino acids, and is a peptide such that the gene coding for said peptide retains the function of enhancing promoter-dependent exogenous protein expression.

18. Silk thread according to any one of claims 1 to 17, wherein the spider thread protein is not fused to a selection marker protein.

19. A transgenic silkworm possessing a pair of fibroin H chain genes and producing silk thread according to any one of claims 1 to 18 wherein the gene coding for spider thread protein is transferred into a region other than the pair of fibroin H chain genes.

20. A transgenic silkworm according to claim 19, **characterized by** having a fibroin H chain gene promoter for expression of spider thread protein in the gene recombinant silkworm.

21. A transgenic silkworm according to claim 19, **characterized by** having a fibroin H chain gene promoter and its upstream region for expression of spider thread protein in the gene recombinant silkworm.

22. A transgenic silkworm according to claim 20 or 21, **characterized in that** the entirety or a portion of the full-length first exon·first intron·second exon region of the fibroin H chain gene is linked downstream from the fibroin H chain promoter.

23. A method for producing a transgenic silkworm according to any one of claims 19 to 22, which utilizes a transposon.

24. A method for producing a transgenic silkworm according to claim 23, **characterized in that** the transposon is piggyBac transposon.

25. A method for producing silk thread **characterized by** using a transgenic silkworm according to any one of claims 19 to 22.

26. A textile employing silk thread according to any one of claims 1 to 18.


SEQUENCE LISTING

<110> TORAY INDUSTRIES, INC.
<110> E. I. DU PONT DE NEMOUS AND COMPANY
<120> SILK THREAD CONTAINING SPIDER SILK THREAD PROTEIN AND SILKWORM PRODUCING SAID
SILK THREAD
<130> FP1085SUBARU
<150> JP 2004-005489
<151> 2004-01-13
<160> 12

<210> 1
<211> 101
<212> PRT
<213> Artificial Sequence
<400> 1
Ser Gln Gly Ala Gly Gln Gly Gly Tyr Gly Gly Leu Gly Ser Gln Gly
1               5                   10                  15
Ala Gly Arg Gly Gly Leu Gly Gly Gln Gly Ala Gly Ala Ala Ala Ala
            20                  25                  30
Ala Ala Ala Gly Gly Ala Gly Gln Gly Gly Leu Gly Ser Gln Gly Ala
        35                  40                  45
Gly Gln Gly Ala Gly Ala Ala Ala Ala Ala Ala Gly Gly Ala Gly Gln
    50                  55                  60
Gly Gly Tyr Gly Gly Leu Gly Ser Gln Gly Ala Gly Arg Gly Gly Gln
65                  70                  75                  80
Gly Ala Gly Ala Ala Ala Ala Ala Ala Gly Gly Ala Gly Gln Gly Gly
                85                  90                  95
Tyr Gly Gly Leu Gly
            100 101

<210> 2
<211> 96
<212> PRT
<213> Artificial Sequence
<400> 2
Ser Gly Gly Ala Gly Gly Ala Gly Gly Ser Gly Gly Ala Gly Gly Ala
1               5                   10                  15
Gly Gly Ser Gly Gly Ala Gly Gly Ala Gly Gly Ser Gly Gly Ala Gly
            20                  25                  30
Gly Ala Gly Gly Ser Gly Pro Gly Gln Gln Gly Pro Gly Gly Tyr Gly
        35                  40                  45
Ser Gly Ala Gly Ala Gly Ser Gly Ala Gly Ala Gly Ser Gly Ala Gly
    50                  55                  60
Ala Gly Ser Gly Ala Gly Ala Gly Ser Gly Ala Gly Ala Gly Ser Gly
65                  70                  75                  80
Ala Gly Ala Gly Ser Gly Ala Gly Ala Gly Ser Gly Ala Gly Ala Gly
                85                  90                  95  96

<210> 3
<211> 33
<212> PRT
<213> Bombyx mori

<400> 3
Arg Ser Tyr Asp Tyr Ser Arg Arg Asn Val Arg Lys Asn Cys Gly Ile
1               5                   10                  15
Pro Arg Arg Gln Leu Val Val Lys Phe Arg Ala Leu Pro Cys Val Asn
            20                  25                  30
Cys
33

<210> 4
<211> 35
<212> PRT
<213> Bombyx mori
<400> 4
Met Arg Val Lys Thr Phe Val Ile Leu Cys Cys Ala Leu Gln Tyr Val
1               5                   10                  15
Ala Tyr Thr Asn Ala Asn Ile Asn Asp Phe Asp Glu Asp Tyr Phe Gly
            20                  25                  30
Ser Asp Val
        35

<210> 5
<211> 27
<212> DNA
<213> Artificial Sequence
<400> 5
aatggcgcgc cgggagaaag catgaag                                          27

<210> 6
<211> 30
<212> DNA
<213> Artificial Sequence
<400> 6
catggatccg acatcactcc caaaatagtc                                       30

<210> 7
<211> 31
<212> DNA
<213> Artificial Sequence
<400> 7
cccaatttgg cgcgcctcaa gacatccttg a                                     31

<210> 8
<211> 27
<212> DNA
<213> Artificial Sequence
<400> 8
gaatgctacc tcgaggttat gaaaatg                                          27

<210> 9
<211> 33
<212> DNA
<213> Artificial Sequence
<400> 9
gctggatccc gcagttacga ctattctcgt cgt                                   33

<210> 10
<211> 35
<212> DNA
<213> Artificial Sequence
<400> 10
cttggcgcgc cacgacgtag acgtatagcc atcgg                    35

<210> 11
<211> 18
<212> PRT
<213> Artificial Sequence
<400> 11
Cys Gly Ala Gly Gln Gly Gly Tyr Gly Gly Leu Gly Ser Gln Ala Gly
1               5                   10                  15
Arg Gly
    18

<210> 12
<211> 19
<212> PRT
<213> Artificial Sequence
<400> 12
Cys Gly Pro Gly Gln Gln Gly Pro Gly Gly Tyr Gly Pro Gly Gln Gln
1               5                   10                  15
Gly Pro Ser
        19